# FASCICULE DE BREVET EUROPEEN

(11) **EP 4 069 221 B1**
(45) Date de publication et mention de la délivrance du brevet: **08.05.2024**
(21) Numéro de dépôt: 20816246.1
(22) Date de dépôt: 04.12.2020
(51) Int. Cl.: A61K 31/43, A61K 36/23, A61K 36/28, A61K 36/54, A61K 36/61, A61K 36/899, A61K 45/06, A61P 31/04, A61P 31/10

(54) **PROCEDE DE TRANSFORMATION D'UNE HUILE ESSENTIELLE**
VERFAHREN ZUR UMWANDLUNG EINES ÄTHERISCHEN ÖLS
METHOD FOR TRANSFORMING AN ESSENTIAL OIL

(30) Priorité: 04.12.2019 EP 19306569
(43) Date de publication de la demande: 12.10.2022
(73) Titulaire: Pranarôm International S.A., 7822 Ghislenghien (BE); Université Libre de Bruxelles, 1050 Bruxelles (BE); Université Grenoble Alpes, 38400 Saint Martin d'Hères (FR); Centre national de la recherche scientifique, 75016 Paris (FR)
(72) Inventeur: ZHIRI, Abdesselam, 1600 Sint-Pieters-Leeuw (BE); OLIVEIRA RIBEIRO, Sofia, 1070 Anderlecht (BE); STÉVIGNY, Caroline, 1560 Hoeilaart (BE); SOUARD, Florence, 64600 Anglet (FR); BAUDOUX, Dominique, 1480 Saintes (BE)
(74) Mandataire: Gevers Patents
(86) Numéro de dépôt international: PCT/EP2020/084631
(87) Numéro de publication internationale: WO 2021/110915

(56) Documents cités:
- WO-A1-01/15680
- WO-A1-2012/023146
- WO-A1-2012/114201
- CN-A- 109 679 767
- FR-A1- 2 918 571
- GRADINARU A C ET AL: "Antibacterial activity of traditional spices against lower respiratory tract pathogens: combinatorial effects of Trachyspermum ammi essential oil with conventional antibiotics", LETTERS IN APPLIED MICROBIOLOGY, vol. 67, no. 5, novembre 2018 (2018-11), pages 449-457, XP055696065,
- EZ ZOUBI Y ET AL: "The antimicrobial activity of moroccan lavaender esssentiel oil against bacterial pathogens isolated urinary tract infectious", INTERNATIONAL JOURNAL OF PHARMACEUTICAL AND CLINICAL RESEARCH 20161101 INTERNATIONAL JOURNAL OF PHARMACEUTICAL AND CLINICAL RESEARCH IND, vol. 8, no. 11, 1 novembre 2016 (2016-11-01), pages 1522-1527, XP055696055, ISSN: 0975-1556
- JULIEN SFEIR ET AL: "In Vitro Antibacterial Activity of Essential Oils against Streptococcus pyogenes", EVIDENCE-BASED COMPLEMENTARY AND ALTERNATIVE MEDICINE, vol. 2013, 1 janvier 2013 (2013-01-01), pages 1-9, XP055664378, US ISSN: 1741-427X, DOI: 10.1155/2013/269161
- TARIQ SAIKA ET AL: "A comprehensive review of the antibacterial, antifungal and antiviral potential of essential oils and their chemical constituents against drug-resistant microbial pathogens", MICROBIAL PATHOGENESIS, ACADEMIC PRESS LIMITED, NEW YORK, NY, US, vol. 134, 11 juin 2019 (2019-06-11), XP085782635, ISSN: 0882-4010, DOI: 10.1016/J.MICPATH.2019.103580 [extrait le 2019-06-11]
- SARA A. BURT ET AL: "Increase in Activity of Essential Oil Components Carvacrol and Thymol against Escherichia coli O157:H7 by Addition of Food Stabilizers", JOURNAL OF FOOD PROTECTION, vol. 68, no. 5, 1 mai 2005 (2005-05-01), pages 919-926, XP055695557, US ISSN: 0362-028X, DOI: 10.4315/0362-028X-68.5.919
- DATABASE EMBASE [Online] ELSEVIER SCIENCE PUBLISHERS, AMSTERDAM, NL; 1 November 2016 (2016-11-01), EZ ZOUBI Y ET AL: "The antimicrobial activity of moroccan lavaender esssentiel oil against bacterial pathogens isolated urinary tract infectious", Database accession no. EMB-20160876407 & EZ ZOUBI Y ET AL: "The antimicrobial activity of moroccan lavaender esssentiel oil against bacterial pathogens isolated urinary tract infectious", INTERNATIONAL JOURNAL OF PHARMACEUTICAL AND CLINICAL RESEARCH 20161101 INTERNATIONAL JOURNAL OF PHARMACEUTICAL AND CLINICAL RESEARCH IND, vol. 8, no. 11, 1 November 2016 (2016-11-01), pages 1522-1527, ISSN: 0975-1556
- DATABASE BIOSIS [Online] BIOSCIENCES INFORMATION SERVICE, PHILADELPHIA, PA, US; November 2018 (2018-11), GRADINARU A C ET AL: "Antibacterial activity of traditional spices against lower respiratory tract pathogens: combinatorial effects of Trachyspermum ammi essential oil with conventional antibiotics", Database accession no. PREV201900036847 & LETTERS IN APPLIED MICROBIOLOGY, vol. 67, no. 5, November 2018 (2018-11), pages 449-457, ISSN: 0266-8254(print), DOI: 10.1111/LAM.13069

## Description

### Domaine technique

La présente invention fournit un procédé de transformation d'une huile essentielle (HE), la fraction d'huile essentielle ainsi obtenue et son utilisation comme médicament, en particulier, son utilisation dans la prévention ou le traitement de maladies infectieuses d'origine bactérienne. La présente invention fournit, en outre, des combinaisons comprenant la fraction d'huile essentielle obtenue selon le procédé de la présente invention avec un antibiotique ainsi que les utilisations de ces combinaisons comme médicament, en particulier, leur utilisation dans la prévention ou le traitement de maladies infectieuses d'origine bactérienne.

### Arrière-plan technologique

Selon les pharmacopées européenne et française, une huile essentielle (HE) est définie comme un « *un produit odorant, généralement de composition complexe, obtenu à partir d'une matière première végétale botaniquement définie, soit par entraînement à la vapeur d'eau, soit par distillation sèche, soit par un procédé mécanique approprié sans chauffage. L'HE est le plus souvent séparée de la phase aqueuse par un procédé physique n'entraînant pas de changement significatif de sa composition».*

Cette définition est également reprise par les normes AFNOR (Association française de normalisation) et ISO (Organisation internationale de normalisation), ainsi que par l'ANSM (Agence nationale de sécurité du médicament et des produits de santé) et les instances internationales.

La plupart des huiles essentielles sont obtenues par distillation par entrainement à la vapeur d'eau. Ce procédé est de loin le plus répandu, car il convient à la majorité des plantes et c'est la méthode validée par la majorité des pharmacopées. Le but est d'emporter avec la vapeur d'eau les constituants les plus volatils. La vapeur détruit la structure des cellules végétales, libère les molécules contenues et entraîne les molécules les plus volatiles en les séparant du substrat cellulosique. La vapeur, chargée de l'essence de la matière première distillée, se condense dans le réfrigérant d'un alambic avant d'être récupérée dans un vase de décantation. Les parties insolubles dans l'eau de condensation sont décantées pour obtenir l'huile essentielle.

Le règne végétal compte plusieurs centaines de milliers d'espèces et 4000 d'entre elles fabriquent des essences aromatiques; dont seulement quelques centaines en quantité suffisante pour qu'on puisse les extraire.

La conservation des huiles essentielles est légalement autorisée pour une durée de 5 ans. Par ailleurs, les essences obtenues par expression à froid de *citrus* ne se conservent que 3 ans.

Les huiles essentielles peuvent être utilisées par voie interne, externe ou aérienne. Administrées par la voie interne, les huiles essentielles sont parfois irritantes pour les muqueuses, on les mélange généralement à de l'huile végétale, à du miel, de la mie de pain ou un support neutre car elles ne se diluent pas dans l'eau. En pharmacie, il est possible d'acheter des préparations de type oléocapsules (avec une base d'huile végétale et capsules gastro-résistantes) ainsi que des préparations sous la forme de gélules, et de suppositoires. L'huile végétale ou l'huile essentielle peuvent pénétrer dans l'organisme à travers la peau (voie externe). Dans ce cas, elle est alors mélangée à une huile végétale de massage ou à une cire, un onguent, une crème ou un lait corporel. Enfin, la diffusion sèche ou humide des huiles essentielles diffusées dans l'air est également utilisée.

Une huile essentielle peut renfermer jusqu'à plusieurs centaines de molécules différentes, dont nombre d'entre elles ont des propriétés particulières. Par exemple, l'huile essentielle de sauge sclarée (*Salvia sclarea*)*,* contient généralement 250 molécules différentes. Ces molécules sont regroupées en plusieurs «familles biochimiques» : cétones, acides, aldéhydes, alcools, esters, ethers, sesquiterpènes, monoterpènes, oxydes aromatiques, hydrocarbures, phénols. Cette « carte d'identité » chimique de l'huile essentielle est le reflet de ses propriétés et diffère en fonction de son espèce ou sous-espèce, son origine géographique, son organe producteur, la nature du sol, l'altitude, la durée d'ensoleillement et son environnement végétal. De nombreuses huiles essentielles possèdent différents chémotypes.

L'utilisation des huiles essentielles à des fins thérapeutiques est appelée l'aromathérapie. Il s'agit d'un domaine de la phytothérapie qui prend de l'ampleur dans le domaine de la santé en tant que thérapie complémentaire. Les huiles essentielles ont déjà démontré, souvent en *in vitro,* des effets antiseptiques, antibactériens, immuno-stimulants, décongestionnants, apaisants, anti-spasmodiques, stimulants ou encore anti-inflammatoires.

L'action antibactérienne (propre ou combinatoire) des huiles essentielles représente un intérêt particulièrement recherché au vu des résistances aux antibiothérapies, problème majeur de santé publique. Les antibiotiques ont permis de faire considérablement reculer la mortalité associée aux maladies infectieuses au cours du 20^{e} siècle. L'efficacité remarquable des antibiotiques a motivé leur utilisation massive et répétée en santé humaine et animale. Toutefois, leur utilisation massive et répétée a conduit à l'apparition de souches bactériennes résistantes. Ponctuelles au départ, ces résistances sont devenues massives et préoccupantes. Certaines souches sont multi-résistantes, c'est-à-dire résistantes à plusieurs antibiotiques, d'une même famille ou de familles différentes. Ce phénomène, en augmentation constante, place les experts de la santé dans une impasse thérapeutique : ils ne disposent plus de solutions pour lutter contre l'infection.

Il est connu de l'état de la technique que les HE possèdent des propriétés antibactériennes. Par exemple, le document DE202007015195U1 divulgue que le linalool, un composé de l'huile essentielle de coriandre séparé par chromatographie sur colonne après une extraction par distillation à la vapeur à partir de graines, possède une activité antimicrobienne vis-à-vis de S. *aureus* (activité propre) ainsi que des propriétés anti-inflammatoires.

L'association de composés d'huiles essentielles avec un antibiotique semble également présenter un certain intérêt car certains de ces composés permettraient de sensibiliser des souches bactériennes résistantes à l'antibiotique. Par exemple, le document WO2006/120494A1 a évalué la sensibilisation de *K. pneumoniae* à l'amoxicilline par le carvéol. Les résultats révèlent que les souris traitées avec du carvéol seul présentent un pourcentage de survie similaire aux souris non traitées et aux souris traitées uniquement avec l'amoxicilline. Au contraire, la combinaison du carvéol et de l'amoxicilline protège les souris traitées. Selon ce document, le carvéol pouvant être issu d'une huile essentielle ou synthétisé chimiquement.

De même, le document WO2017/209588A2 divulgue une composition pharmaceutique comprenant du 1,8-cinéol et de l'amoxicilline. Selon ce document, le 1,8-cinéol potentialise l'effet de l'amoxicilline et sensibilise des souches bactériennes, *E.coli et K. pneumoniae,* initialement résistantes. Le cinéol utilisé dans le document WO2017/209588A2 est du cinéol commercial. Ce document mentionne que le cinéol peut être extrait de certains eucalyptus, de romarins, de l'armoise, d'absinthes, de lauriers, de sauges, du basilic et de camphrier.

Malheureusement, les compositions antibactériennes à base d'huiles essentielles qui existent actuellement, en particulier les compositions antibactériennes des documents DE202007015195U1, WO2006/120494A1 et WO2017/209588A2 présentent des limitations. En particulier, il est encore nécessaire d'accroitre l'activité antimicrobienne des compositions à base d'huiles essentielles, vis-à-vis d'un nombre acceptable de souches bactériennes et fongiques. Par ailleurs, il est également nécessaire de développer de nouvelles compositions à base d'huiles essentielles qui permettent d'accroitre d'avantage l'effet potentialisateur d'agents antimicrobiens, tels que des antibiotiques ou des antifongiques.

### Description de l'invention

L'invention permet de résoudre les inconvénients de l'état de la technique en procurant un procédé de transformation d'une huile essentielle qui conduit à l'obtention d'une huile essentielle transformée, correspondant à la fraction non-évaporée de l'huile essentielle. L'huile essentielle transformée par le procédé selon l'invention présente une action antibactérienne améliorée par rapport à une huile essentielle non transformée, possède une activité propre et une activité combinatoire (en association avec différents antibiotiques) et possède une action à large spectre antibactérien.

Pour résoudre ce problème, il est prévu suivant l'invention un procédé de transformation d'une huile essentielle comprenant :
- fournir une huile essentielle préalablement extraite;
- évaporer une fraction comprise entre 20 et 50% de l'huile essentielle; et
- obtenir une huile essentielle transformée comprenant la récupération de la fraction non-évaporée de l'huile essentielle,
- combiner ladite fraction non-évaporée avec au moins un antibiotique;
caractérisé en ce que l'évaporation est réalisée durant au moins 2 h, sous une pression inférieure ou égale à 500 mbar et à une température comprise entre 40 et 100 °C, et en ce que l'huile essentielle est préalablement extraite de l'ajowan, de la camomille allemande, du lemongrass, du palmarosa, de la lavande stoechade, de l'origan compact ou de la cannelle de Chine.

Dans un mode de réalisation préféré de l'invention, une HE fait référence à une HE telle que définie selon les pharmacopées européenne et française, à savoir définie comme *un produit odorant, généralement de composition complexe, obtenu à partir d'une matière première végétale botaniquement définie, soit par entraînement à la vapeur d'eau, soit par distillation sèche, soit par un procédé mécanique approprié sans chauffage. L'HE est le plus souvent séparée de la phase aqueuse par un procédé physique n'entraînant pas de changement significatif de sa composition.*

Selon un mode de réalisation particulier de l'invention, l'HE est un produit odorant obtenu à partir d'une matière première végétale botaniquement définie, soit par entraînement à la vapeur d'eau, soit par distillation sèche, soit par un procédé mécanique approprié sans chauffage. De manière préférée, l'HE selon l'invention est obtenue par entraînement à la vapeur d'eau.

Préférentiellement, l'HE selon l'invention n'est pas obtenue par une méthode autre que la distillation ou le pressage à froid, par exemple, n'est pas obtenue par une extraction avec un solvant, par une extraction par fluide supercritique, par une double distillation ou par une distillation et une extraction simultanées.

Dans le cadre de la présente invention, toutes les quantités définies en pourcentages (%) font référence à des pourcentages volumiques (% volumique, % volume/volume, % v/v), à moins que le contexte n'indique clairement le contraire. Il sera donc par exemple clair pour l'homme du métier que l'évaporation de 7 % d'HE signifie que 7 % du volume initial d'HE s'est évaporé, tandis que 93 % du volume initial d'HE ne s'est pas évaporé. Toujours selon cet exemple, l'HE transformée obtenue par le procédé selon l'invention correspond donc à la fraction de l'HE qui ne s'est pas évaporée, à savoir 93% du volume initial d'HE. De même, l'évaporation de 90% de l'HE signifie que 90% du volume initial d'HE s'est évaporé, tandis que 10% du volume initial d'HE ne s'est pas évaporé. Toujours selon cet exemple, l'HE transformée obtenue par le procédé selon l'invention correspond donc à la fraction de l'HE qui ne s'est pas évaporée, à savoir 10% du volume initial d'HE.

Les inventeurs ont observé de manière surprenante que le procédé selon l'invention permettait d'obtenir une huile essentielle transformée qui présente une activité antibactérienne améliorée. Par ailleurs, l'huile essentielle transformée par le procédé selon l'invention est utilisée en association avec un antibiotique, et est efficace contre différents types bactériens.

Dans le cadre de la présente invention, l'activité propre fait référence à l'activité antimicrobienne exercée par l'HE elle-même. L'activité propre peut, par exemple, être évaluée simplement en mesurant la CMI (concentration minimale inhibitrice), qui est la plus petite concentration suffisante pour inhiber, *in vitro,* la croissance d'une souche bactérienne, après traitement de différentes souches bactériennes par différentes HE transformées ou non selon le procédé de l'invention.

Il a été observé que les HE transformées selon le procédé de l'invention présentent une activité propre améliorée, à savoir une diminution de la CMI, sur différentes souches bactériennes par rapport à une HE non transformée selon le procédé de l'invention. Par exemple, l'HE d'ajowan et l'HE de la lavande stoechade transformées selon le procédé de l'invention présentent une activité au moins 2 fois supérieure sur au moins 3 souches bactériennes.

L'activité combinatoire fait référence à l'interaction synergétique observée entre l'HE transformée et un agent antimicrobien (qui n'est pas partie de l'huile essentielle). L'activité combinée peut être évaluée par la méthode de microdilution. Par exemple, par une méthode de microdilution en plaque de 96 puits où les produits seuls et/ou combinés sont dilués par deux, à partir d'une concentration initiale de l'ordre de 1000 µg/ml. Par cette méthode on détermine la CMI. Une amélioration de l'activité est observée quand la CMI de l'association d'un antibiotique avec l'huile essentielle transformée ou non est inférieure à celle de l'antibiotique seul. Par exemple, si la CMI de l'antibiotique seul est de 4 µg/ml et que la valeur obtenue en activité combinatoire est de 0,5 µg/ml, il y a une amélioration de l'activité de 8 x l'activité de l'antibiotique seul. Plus la diminution de la CMI est importante, plus la concentration nécessaire d'antibiotique est faible lorsqu'il est associé à l'HE. Plus la CMI est diminuée, plus l'activité de l'antibiotique vis-à-vis de la souche bactérienne est potentialisée. En diminuant les concentrations de l'huile essentielle en deçà de sa CMI il est vraisemblable que l'on ait des valeurs non toxiques.

En particulier, les inventeurs ont observé une activité combinatoire améliorée, à savoir une diminution de la CMI observée avec l'association quand comparée à celle observée avec l'antibiotique seul lorsque les bactéries sont traitées avec différentes HE transformées selon le procédé de l'invention. A titre d'exemple, l'HE d'ajowan (*Trachyspermum ammi*)*,* l'HE de la camomille allemande (*Matricaria recutita*)*,* l'HE de lemongrass (*Cymbopogon citratus*) et l'HE de la lavande stoechade (*Lavandula stoechas*)*,* sensibilisent 3 souches bactériennes normalement résistantes à plusieurs antibiotiques, lorsqu'elles sont transformées par le procédé selon l'invention.

Selon l'invention, l'évaporation est comprise entre 20 et 50 %, préférentiellement comprise entre 30 et 50 %, par exemple d'environ 45 % de l'huile essentielle. En effet, il a été observé que l'activité propre et combinatoire était améliorée de manière croissante en fonction du pourcentage d'évaporation de l'HE transformée selon le procédé de l'invention. Par exemple, en activité combinatoire avec la Pénicilline V, l'HE de l'origan compact (*Origanum compactum*) transformée selon le procédé de l'invention montre, sur la souche S. *aureus* LMG 15975, une CMI de 0,125 µg/ml pour une évaporation d'environ 16 % et diminue à une CMI de 0,03 µg/ml pour une évaporation d'environ 27,5 %. En activité propre, dans le cas d'une HE de lavande stoechade transformée selon l'invention, les CMI calculées pour la souche S. *aureus* LMG 8064 diminuent entre l'HE non transformée et transformée selon l'invention, mais diminuent également encore de moitié lorsque l'évaporation passe de 19 à 24 % et encore de moitié quand l'évaporation passe de 24 à 41,5 %.

Le procédé selon l'invention comprend les étapes de fournir une huile essentielle préalablement extraite, évaporer entre 20 et 50 % de l'huile essentielle, et obtenir une huile essentielle transformée comprenant la récupération de la fraction non-évaporée de l'huile essentielle. De manière préférée, le procédé selon l'invention comprend les étapes de fournir une huile essentielle préalablement extraite, évaporer entre 30 et 50 % de l'huile essentielle, et obtenir une huile essentielle transformée comprenant la récupération de la fraction non-évaporée de l'huile essentielle. Par exemple, le procédé selon l'invention comprend les étapes de fournir une huile essentielle préalablement extraite, évaporer environ 45 % de l'huile essentielle, et obtenir une huile essentielle transformée comprenant la récupération de la fraction non-évaporée de l'huile essentielle.

Avantageusement, l'évaporation est réalisée pendant une durée comprise entre 2 et 360 h, préférentiellement comprise entre 2 et 240 h, de préférence comprise entre 3 et 96 h, encore préférentiellement comprise entre 18 et 96 h, encore plus préférentiellement comprise entre 24 et 96h, par exemple pendant une durée d'environ 72 h. Le pourcentage d'évaporation va, notamment, dépendre de la durée de l'évaporation et de la volatilité de chaque HE étudiée. Par exemple, pour une HE faiblement volatile, comme l'HE de palmarosa (*Cymbopogon martinii var. motia*)*,* une diminution de la CMI est déjà observée après 3h.

Dans un mode particulier de l'invention, l'évaporation est réalisée sous une pression inférieure ou égale à 300 mbar, de préférence inférieure ou égale à 150 mbar, encore préférentiellement inférieure ou égale à 100 mbar, encore plus préférentiellement comprise entre 25 et 75 mbar, par exemple d'environ 60 mbar.

De préférence, l'évaporation est réalisée à une température comprise entre 40 et 60 °C, par exemple à une température d'environ 40 °C.

Selon un mode préféré de l'invention, l'huile essentielle préalablement extraite est évaporée en présence d'une solution comprenant un solvant choisi parmi les hydrocarbures comme le chloroforme, le dichlorométhane, le n-hexane, le cyclohexane ou l'éther de pétrole ; les éthers comme l'éther diéthylique ; les esters comme l'acétate d'éthyle ; les cétones comme l'acétone ; les alcools comme le méthanol, l'éthanol ou l'isopropanol ; et de leurs mélanges. Selon ce mode particulier de l'invention, la solution comprenant le solvant aide à l'évaporation de l'HE. Elle est ajoutée avant l'évaporation et est substantiellement complètement évaporée durant l'évaporation d'une fraction de l'HE.

Préférentiellement, la solution comprenant un solvant est un mélange hydro-alcoolique, l'alcool étant présent à hauteur d'au moins 50 %.

Avantageusement, l'évaporation est réalisée sous agitation, de préférence sous une agitation comprise entre 20 et 300 rotations/minute, préférentiellement comprise entre 200 et 300 rotations/minute.

Selon l'invention, l'huile essentielle est préalablement extraite d'une plante sélectionnée dans le groupe constitué de l'ajowan (*Trachyspermum ammi*)*,* de la camomille allemande (*Matricaria recutita*)*,* de la cannelle de Chine (*Cinnamomum cassia*), de la lavande stoechade (*Lavandula stoechas*), de lemongrass (*Cymbopogon citratus*), de l'origan compact (*Origanum compactum*), de la palmarosa (*Cymbopogon martinii var. motia*), et de leurs mélanges.

Selon l'invention, la camomille est la camomille allemande, la cannelle est la cannelle de Chine, la lavande est la lavande stoechade.

Dans un mode de réalisation préféré de l'invention, l'huile essentielle est préalablement extraite de la lavande stoechade ou de l'origan compact, de préférence l'huile essentielle est préalablement extraite de la lavande stoechade. En effet, les inventeurs ont observé que l'action antibactérienne était particulièrement efficace lorsque la plante à partir de laquelle l'HE est extraite puis transformée selon le procédé de l'invention était l'origan compact ou la lavande stoechade, les résultats étant encore supérieurs avec la lavande stoechade.

Préférentiellement, l'huile essentielle est préalablement extraite de la lavande stoechade et en ce que l'évaporation est réalisée pendant une durée comprise entre 24 et 96 h, sous une pression comprise entre 25 et 75 mbar et à une température comprise entre 40 et 60 °C. Les inventeurs ont, effectivement, observé que l'action antibactérienne était particulièrement améliorée lorsque l'HE était extraite de la lavande stoechade et transformée selon le procédé de l'invention sous ces paramètres particuliers.

La fraction d'huile essentielle selon l'invention correspond à la fraction non-évaporée de l'huile essentielle obtenue par une transformation selon le procédé de l'invention. L'HE est d'abord préalablement extraite. Les méthodes d'extraction de l'HE peuvent être toutes les méthodes d'extraction d'HE connues de l'homme de métier. De préférence, les méthodes d'extraction de l'HE sont celles qui permettent d'obtenir une HE telle que définie selon les pharmacopées européenne et française, à savoir par distillation par entrainement à la vapeur d'eau, par distillation sèche ou par un procédé mécanique approprié sans chauffage. En particulier, l'HE est extraite par distillation par entrainement à la vapeur d'eau. Une fois l'HE extraite, une évaporation d'une fraction comprise entre 20 et 50% de l'HE est réalisée durant au moins 2 h, sous une pression inférieure ou égale à 500 mbar et à une température comprise entre 40 et 100 °C. L'HE essentielle ainsi transformée comprend la fraction non-évaporée de l'HE.

La fraction évaporée de l'HE correspond à un pourcentage compris entre 20 et 50% de l'HE et la fraction d'HE selon l'invention, à savoir la fraction non évaporée de l'HE obtenue par le procédé selon l'invention correspond à un pourcentage compris entre 50 et 80% de l'HE extraite. Une fraction d'HE selon l'invention, à savoir la fraction d'HE non évaporée correspondant à un pourcentage compris entre 50 et 80% de l'HE possède une activité propre et/ou combinatoire qui est améliorée.

La fraction l'HE non évaporée selon l'invention correspond à un pourcentage compris entre 50 et 80 %, préférentiellement compris entre 50 et 70 %, de manière entre plus préférée d'environ 55 % de l'HE. En effet, il a été observé que la fraction d'HE non évaporée selon l'invention présente une activité antibactérienne qui s'améliore proportionnellement avec la diminution du pourcentage de la fraction d'HE non évaporée selon l'invention.

Selon l'invention, la fraction non évaporée d'HE selon l'invention est issue d'une plante sélectionnée dans le groupe constitué de l'ajowan, de la camomille, de la cannelle, de la lavande, de la lemongrass, de l'origan, de la palmarosa, et de leurs mélanges.

Selon l'invention, la camomille est la camomille allemande, la cannelle est la cannelle de Chine, la lavande est la lavande stoechade.

Dans un mode de réalisation préféré de l'invention, la fraction non évaporée de l'HE selon l'invention est obtenue d'une huile essentielle préalablement extraite de la lavande stoechade ou de l'origan compact, de préférence l'huile essentielle est préalablement extraite de la lavande stoechade. En effet, les inventeurs ont observé que l'action antibactérienne était particulièrement efficace lorsque la fraction non évaporée de l'HE selon l'invention est transformée à partir d'une HE extraite de l'origan compact ou la lavande stoechade, les résultats étant encore supérieurs avec la lavande stoechade.

Préférentiellement, la fraction non évaporée de l'HE selon l'invention est transformée à partir d'une huile essentielle qui est préalablement extraite de la lavande stoechade, la transformation comprenant une évaporation pendant une durée comprise entre 24 et 96 h, sous une pression comprise entre 25 et 75 mbar et à une température comprise entre 40 et 60 °C. Les inventeurs ont, effectivement, observé que l'action antibactérienne était particulièrement améliorée avec une telle fraction non évaporée d'HE selon l'invention.

Un autre objet de l'invention est également une combinaison comprenant une fraction d'huile essentielle selon l'invention et une seconde huile essentielle qui a été transformée ou non par le procédé selon l'invention.

Un autre aspect de l'invention est une combinaison comprenant une fraction d'huile essentielle selon l'invention et au moins un antibiotique.

De préférence, l'antibiotique est sélectionné dans le groupe constitué des beta-lactames, des tétracyclines, des aminoglycosides, des antibiotiques acides phosphoniques, des macrolides, des quinolones, etc et de leurs mélanges.

Il a été observé que l'activité combinatoire est améliorée, c'est-à-dire qu'une diminution de la CMI de l'association d'un antibiotique avec la fraction non évaporée d'HE selon l'invention est observée, par rapport à la CMI de l'antibiotique seul.

Préférentiellement, l'huile essentielle est préalablement extraite de la lavande stoechade et l'antibiotique est une pénicilline. L'effet antibactérien est particulièrement efficace lorsque la fraction non évaporée de l'invention est issue de la lavande stoechade et est combinée avec une pénicilline.

La présente invention porte également sur l'utilisation de la fraction et des combinaisons selon l'invention comme médicament.

Dans le cadre de la présente invention, la fraction et les combinaisons selon l'invention peuvent également être utilisées en cosmétique ou peuvent concerner le domaine de la médecine humaine ou vétérinaire.

La présente invention porte aussi sur l'utilisation de la fraction et des combinaisons selon l'invention dans la prévention ou le traitement de maladies infectieuses d'origine bactérienne.

De préférence, la bactérie à l'origine de la maladie infectieuse est sélectionnée dans le groupe constitué de la famille *Staphylococcaceae* (ex : *Staphylococcus aureus*)*,* de la famille *Enterobacteriaceae* (ex : *Escherichia coli, Enterobacter aerogenes* et *Klebsiella pneumoniae*), de la famille *Pseudomonadaceae* (ex : *Pseudomonas aeruginosa*), de la famille *Mycobacteriaceae* (ex : *Mycobacterium bovis*), de la famille *Enterococcaceae* (ex : *Enterococcus faecalis*), de la famille *Helicobacteriaceae* (ex : *Helicobacter pylori*), de la famille des *Streptococcaceae* (ex : *Streptococcus mutans*), de la famille *Moraxellaceae* (ex : *Acinetobacter baumannii*), de la famille *Clostridiaceae* (ex : *Clostridium difficile*), de la famille *Spirochaetaceae* (ex : *Borrelia garinii*) et de leurs mélanges.

Selon un mode de réalisation préféré de l'invention, l'huile essentielle est préalablement extraite de la lavande stoechade et la bactérie est S. *aureus.*

Ces modes de réalisation ainsi que d'autres modes de réalisation de la présente invention sont indiqués dans les revendications annexées.

### Exemples

L'invention va maintenant être décrite plus en détails dans les exemples suivants qui décrivent des modes de réalisations non limitatifs de différents aspects de la présente invention.

### 1. Procédé de transformation des huiles essentielles (HE)

Des HE commerciales (Pranarom) d'ajowan (AW), de basilic exotique (BE), de camomille allemande (CA), de cannelle de Chine (CC), de coriandre feuille (CF), de giroflier (GF), de citronnelle (LG), de lavande stoechade (LS), d'origan compact (OC) et de palmarosa (PM) ont été transformées selon le procédé de l'invention. Pour ce faire, les HE ont été préparées en ajoutant 0,5 ml de chloroforme à 200 µL d'HE. Les tubes ont ensuite été placés dans un évaporateur Syncore^{®} (Büchi) permettant une évaporation contrôlée de 96 échantillons pour une durée définie. L'évaporation des échantillons a été réalisée à une pression de 60 mbar, une température de 40 °C et sous une agitation de 250 rotations/minute.

### 2. Evolution de l'évaporation des HE transformées en fonction du temps

L'évaporation des HE transformées selon le procédé de l'invention tel que décrit ci-dessus, en fonction du temps d'évaporation a été mesurée et analysée par chromatographie gazeuse. Le pourcentage d'évaporation des échantillons a été mesuré après une évaporation de 3 h (T1), 18 h (T2), 24 h (T3), 48 h (T4) et de 72 h (T5). Les résultats, exprimés en pourcentages d'évaporation, sont repris dans les tableaux 1 (pour les HE les moins volatiles) et 2 (pour les HE les plus volatiles).

**Tableau 1 :**

| | **T1** | **T2** | **T3** | **T4** | **T5** |
|---|---|---|---|---|---|
| **CA** | 1,4 | 3,3 | 4,6 | 5,4 | 7,6 |
| **CC** | 1,4 | 2,8 | 3,4 | 4,3 | 5,1 |
| **GF** | 0,7 | 1,3 | 1,8 | 2,8 | 3,3 |
| **LG** | 0,8 | 4,9 | 6,1 | 9,6 | 10,9 |
| **PM** | 0,8 | 1,8 | 2,1 | 3,2 | 3,5 |

| | | | | | |
|---|---|---|---|---|---|
| Légende : les résultats sont exprimés en pourcentages d'évaporation | | | | | |

**Tableau 2 :**

| | **T1** | **T2** | **T3** | **T4** | **T5** |
|---|---|---|---|---|---|
| **AW** | 8,0 | 23,0 | 23,8 | 48,3 | 52,4 |
| **BE** | 2,4 | 6,2 | 7,6 | 13,6 | 18,4 |
| **CF** | 5,4 | 9,9 | 12,8 | 18,3 | 22,9 |
| **LS** | 7,1 | 18,7 | 23,9 | 41,5 | 51,3 |
| **OC** | 6,5 | 15,3 | 15,8 | 24,4 | 27,4 |

| | | | | | |
|---|---|---|---|---|---|
| Légende : les résultats ont exprimés en pourcentages d'évaporation | | | | | |

### 3. Activités propre et combinatoire de fractions non évaporées d'HE transformées selon le procédé de l'invention

L'activité propre d'HE transformées selon le procédé de l'invention (HE-T) avec une évaporation de 3 h (T1), 18 h (T2), 24 h (T3), 48 h (T4) et de 72 h (T5) a été comparée à l'activité propre de l'HE commerciale correspondante non transformée selon le procédé de l'invention (HE-C). Les pourcentages d'évaporation de chaque HE testée en fonction du temps d'évaporation sont présentés dans les tableaux 1 et 2 ci-dessus.

Pour ce faire, les solutions stock d'HE (HE-C et HE-T) ont été préalablement diluées dans du DMSO, à raison de 10 µL d'HE dans 300 µL de DMSO. Elles ont été agitées et stockés à température ambiante, à l'abri de la lumière, dans des tubes en verre autoclavés avec bouchon à vis. L'activité propre des HE a été mesurée par la méthode de micro dilution en plaque 96 puits, de manière à obtenir la CMI, valeur à laquelle une solution inhibe la croissance bactérienne. Les souches bactériennes traitées sont S. *aureus* LMG 15975 staphylocoque doré résistant à la méticilline, ici nommé MRSA +), *E. coli* LMG 8223, *E. coli* LMG 15862, *P. aeruginosa* LMG 6395, *S*. *aureus* LMG 8064, *S*. *aureus* LMG 16217 (staphylocoque doré résistant à la méticilline, ici nommé MRSA ++), *E. faecalis* LMG 8222, *K. pneumoniae* LMG 20218 et *E. aerogenes* LMG 2094. Des inocula des souches bactériennes ont été préparés en plaçant des colonies de 18-24h des souches en suspension dans une solution physiologique stérile (NaCl 0,85 %). Ces inocula ont ensuite été ajustés et dilués de manière à obtenir une concentration finale de 5 × 10⁵ CFU/mL. Concernant les solutions à tester, 300 µL des solutions stocks d'HE (diluées dans du DMSO) ont été ajoutés à 4,7 mL de milieu de culture (par exemple à du *Mueller Hinton Broth*), de manière à obtenir une solution d'HE de 2 mg/mL qui est ensuite diluée en série par deux dans des plaques multipuits. Le pourcentage final de DMSO dans les puits est inférieur à 5%. Après inoculation, les plaques multipuits ont été incubées 18-24 h à 37 °C et la présence de bactéries a été visualisée par l'ajout de méthyltétrazolium (MTT).

L'activité combinatoire permet de déterminer si l'HE ou l'HE-T inhibe la croissance bactérienne à une concentration dite sub-CMI, c'est-à-dire à une concentration à laquelle l'HE ne possède pas d'activité propre. La concentration sub-CMI d'une HE est égale au minimum à deux CMI (dilutions en série par deux) en dessous de laquelle l'HE est active (activité propre). Par exemple, si la CMI d'une HE donnée est 500 µg/mL, la sub-CMI testée sera de minimum 125 µg/mL. Les valeurs de sub-CMI sont reprises dans la colonne HE _{sub-CMI}.

L'activité combinatoire d'HE transformées selon le procédé de l'invention avec une évaporation de 24 h (HE-T3) et de 72 h (HE-T5) a été comparée à l'activité combinatoire de l'HE commerciale correspondante non transformée selon le procédé de l'invention (HE-C). La même méthode de microdilution que celle utilisée pour déterminer l'activité antibactérienne propre a été utilisée pour déterminer l'activité combinatoire. Les antibiotiques testés en association avec les HE transformées ou non sont l'amoxicilline (AMOX), la pénicilline G (PG), la pénicilline V (PV), la céfotaxime (CEFO), la gentamicine (GENTA), la tétracycline (TETRA), la doxycycline (DOXY), l'ampicilline (AMPI) et la fosfomycine (FOSFO). Les types bactériens traités sont *S*. *aureus* LMG 15975 (MRSA+), *S*. *aureus* 16217 (MRSA++), *E. faecalis* LMG 8222, *E. coli* LMG 15862, *P. aeruginosa* LMG 6395, *K. pneumoniae* LMG 20218 et *E. aerogenes* LMG 2094. La différence réside dans le fait que ce sont les antibiotiques qui ont été dilués en série par 2. Les HE, à leur concentration sub-CMI ont ensuite été ajoutées, et les solutions ont été inoculées avec les souches bactériennes précitées.

### Résultats

Les résultats concernant l'activité propre sont exprimés en µg/ml et indiquent la CMI de l'HE lorsqu'elle est transformée ou non par le procédé selon l'invention. Les résultats concernant l'activité combinatoire sont également exprimés en µg/ml et indiquent les valeurs sub-CMI de l'HE, seule ou en combinaison avec l'antibiotique, transformée ou non par le procédé selon l'invention.

Tous les tests *in vitro* sont réalisés au minimum en triplicat sur au moins 3 échantillons différents d'une HE-T.

Le tableau 3 reprend les résultats de l'activité propre et combinatoire en association avec la PV des HE-T selon l'invention d'AW, de LS et d'OC sur la souche *S*. *aureus* LMG 15975. L'antibiotique est la pénicilline V (PV) dont la CMI sur cette souche est de 4 µg/ml.

**Tableau 3 :**

| | | Souche de référence : | | |
|---|---|---|---|---|
| | | *Staphylococcus aureus* LMG 15975 | | |
| | **% d'évaporation** | **Activité propre** | HE_{sub-CMI} (µg/ml) | **Activité combinatoire** |
| | | CMI : PV/HE (µg/ml) | | HE_{sub-CMI} + PV (µg/ml) |
| PV | | 4 | | |
| AW-C | 0 | 500 | 125 | 0,5 |
| AW-T3 | 23,8 | 500 | 125 | 0,125 |
| AW-T5 | 52,4 | 250 | 62,5 | 0,06 |
| LS-C | 0 | > 1000 | 500 | 0,06 |
| LS-T3 | 23,9 | 1000 | 250 | 0,06 |
| LS-T5 | 51,3 | 500 | 125 | 0,03 |
| OC-C | 0 | 250 | 62,5 | 0,25 |
| OC-T3 | 15,8 | 250 | 62,5 | 0,125 |
| OC-T5 | 27,4 | 250 | 62,5 | 0,03 |

Dans le cas de l'ajowan (AW), 50 % des constituants les moins volatils sont présents dans l'HE-T après 72h d'évaporation. Cette transformation, dénommée AW-T5, augmente légèrement l'activité propre de l'ajowan qui passe d'une CMI de 500 µg/ml à une CMI de 250 µg/ml. En activité combinatoire, l'ajowan non transformé (AW-C) a augmenté l'activité de la pénicilline V en diminuant sa CMI qui passe de 4 µg/ml (PV seule) à 0,5 µg/ml (PV + AW-C à 125 µg/ml). L'effet combinatoire est d'autant plus efficace avec les HE-T de AW. Combinée aux HE-T3 (24h d'évaporation) et T5 (72h d'évaporation), la CMI de la pénicilline V diminue à 0,125 µg/ml et à 0,06 µg/ml, respectivement, par rapport à la CMI de la pénicilline V seule qui est de 4 µg/ml.

Dans le cas de la lavande stoechade (LS), les pourcentages d'évaporation sont similaires à ceux de l'ajowan, à savoir qu'à 72h (LS-T5) on atteint également une évaporation d'environ 50% des composés les plus volatils. En activité propre, la CMI de LS-C qui était supérieure à 1000 µg/ml diminue au fur et à mesure jusqu'à atteindre 500 µg/ml à 72h d'évaporation (LS-T5). En activité combinatoire, l'HE-T5 augmente l'activité de la pénicilline V : la CMI de la pénicilline V qui seule est active à 4 µg/ml diminue à 0,06 µg/m! quand elle est associée à LS-C et LS-T3 et à 0,03 µg/ml quand elle est associée à l'HE-T5.

Dans le cas de l'origan (OC), les transformations provoquent une évaporation des composés les plus volatils d'environ 16% à 24h (OC-T3) et d'environ 27% à 72h (OC-T5). L'activité directe des HE-T d'OC est maintenue à 250 µg/ml tout au long des transformations (OC-T3 et OC-T5). En activité combinatoire avec la pénicilline V, les HE-T issues de la transformation ont fortement diminué les CMI par rapport à celle de l'origan commercial (OC-C) : la CMI de la pénicilline V seule est de 4 µg/ml et diminue à 0,25 µg/ml quand elle est associée à OC-C. La CMI de la pénicilline V diminue progressivement à 0,125 µg/ml quand elle est associée à l'extrait OC-T3 et atteint la concentration de 0,03 µg/ml quand elle est associée à OC-T5.

Dans les trois cas, l'association des HE-T5 (AW-T5, LS-T5 et OC-T5) à la pénicilline V permet de descendre à des gammes de concentrations proches de celles obtenues sur la souche MSSA LMG 8064 (*Staphylococcus aureus* sensible à la méticilline) dont la CMI de PV est de 0,015 µg/ml. Les HE-T selon l'invention ont donc la capacité de rétablir l'activité de la pénicilline V sur une souche devenue résistante.

En conclusion, Le procédé de transformation selon l'invention permet d'obtenir des HE-T issues d'huiles essentielles qui ont une activité antibactérienne accrue que ce soit en activité propre quand l'HE-T est utilisée seule et/ou en activité combinatoire quand l'HE-T est combinée à un antibiotique. Ce dernier cas est d'autant plus intéressant car il permettrait de diminuer les doses d'antibiotiques à administrer en les associant à une HE-T dont la dose est non active *perse.*

Le tableau 4 reprend les résultats de l'activité combinatoire en association avec l'AMOX, la PV et la PG de l'HE-T selon l'invention de LS sur la souche *S*. *aureus* LMG 16217. Les antibiotiques sont l'amoxicilline dont la CMI sur cette souche est de 64 µg/ml, la pénicilline G dont la CMI sur cette souche est de 64 µg/ml et la pénicilline V dont la CMI sur cette souche est de 64 µg/ml.

**Tableau 4 :**

| | **% d'évaporation** | Souche de référence : | |
|---|---|---|---|
| | | *Staphylococcus aureus* LMG 16217 | |
| | | HE_{sub-CMI} | **Activité combinatoire** |
| | | (µg/ml) | HE_{sub-CMI} + AB (µg/ml) |
| AMOX | | | |
| LS-C | 0 | 500 | 32 |
| LS-T3 | 23,9 | 125 | 4 |
| LS-T5 | 51,3 | 125 | 4 |

| PV | | | |
|---|---|---|---|
| LS-C | 0 | 500 | 32 |
| LS-T3 | 23,9 | 125 | 4 |
| LS-T5 | 51,3 | 125 | 4 |

| PG | | | |
|---|---|---|---|
| LS-C | 0 | 500 | 32 |
| LS-T3 | 23,9 | 125 | 8 |
| LS-T5 | 51,3 | 125 | 8 |

Le tableau 5 reprend les résultats de l'activité propre des HE-T selon l'invention de AW, CC, LG, OC et PM sur la souche *Escherichia coli* LMG 15862.

**Tableau 5 :**

| | **% d'évaporation** | Souche de référence :*Escherichia coli* LMG 15862 |
|---|---|---|
| | | **Activité propre** |
| | | CMI : HE(µg/ml) |
| AW-C | 0 | 1000 |
| AW-T2 | 23 | 500 |
| AW-T3 | 23,8 | 500 |
| AW-T4 | 48,3 | 250 |
| CC-C | 0 | 500 |
| CC-T2 | 2,8 | 500 |
| CC-T3 | 3,4 | 250 |
| CC-T4 | 4,3 | 250 |
| LG-C | 0 | >1000 |
| LG-T2 | 4,9 | >1000 |
| LG-T3 | 6,1 | >1000 |
| LG-T4 | 9,6 | 1000 |
| OC-C | 0 | 500 |
| OC-T2 | 15,3 | 500 |
| OC-T3 | 15,8 | 250 |
| OC-T4 | 24,4 | 250 |
| PM-C | 0 | >1000 |
| PM-T2 | 1,8 | 1000 |
| PM-T3 | 2,1 | 1000 |
| PM-T4 | 3,2 | 1000 |

Dans tous les cas (AW, CC, LG, OC et PM), l'activité propre augmente lorsque les HE sont transformées selon le procédé de l'invention par rapport aux HE non transformées (HE-C). En effet, on observe une diminution générale de la CMI lorsque la souche *E. coli* LMG 15862 est traitée avec les différentes (AW, CC, LG, OC et PM) HE-T transformées selon le procédé de l'invention.

## Revendications

1. Un procédé de transformation d'une huile essentielle comprenant :
- fournir une huile essentielle préalablement extraite ;
- évaporer une fraction comprise entre 20 et 50% de l'huile essentielle ; et
- obtenir une huile essentielle transformée comprenant la récupération de la fraction non-évaporée de l'huile essentielle ;
- combiner ladite fraction non-évaporée avec au moins un antibiotique,
**caractérisé en ce que** l'évaporation est réalisée durant au moins 2 h, sous une pression inférieure ou égale à 500 mbar et à une température comprise entre 40 et 100 °C, et **en ce que** l'huile essentielle est préalablement extraite de l'ajowan, de la camomille allemande, du lemongrass, du palmarosa, de la lavande stoechade, de l'origan compact ou de la cannelle de Chine.

2. Le procédé de transformation selon la revendication 1, **caractérisé en ce que** l'évaporation est comprise entre 30 et 50 %, de manière préférée est d'environ 45 % de l'huile essentielle.

3. Le procédé de transformation selon la revendication 1 ou 2, **caractérisé en ce que** l'évaporation est réalisée pendant une durée comprise entre 18 et 96 h, encore plus préférentiellement comprise entre 24 et 96h, de manière encore plus préférée pendant une durée d'environ 72 h.

4. Le procédé de transformation selon l'une quelconque des revendications précédentes, **caractérisé en ce que** l'évaporation est réalisée sous une pression inférieure ou égale à 300 mbar, de préférence inférieure ou égale à 150 mbar, encore préférentiellement inférieure ou égale à 100 mbar, encore plus préférentiellement comprise entre 25 et 75 mbar, de manière encore plus préférée d'environ 60 mbar.

5. Le procédé de transformation selon l'une quelconque des revendications précédentes, **caractérisé en ce que** l'évaporation est réalisée à une température comprise entre 40 et 60 °C, encore préférentiellement à une température d'environ 40 °C.

6. Le procédé de transformation selon l'une quelconque des revendications précédentes, **caractérisé en ce que** l'huile essentielle est préalablement extraite de la lavande stoechade ou de l'origan compact, de préférence l'huile essentielle est préalablement extraite de la lavande stoechade.

7. Le procédé de transformation selon l'une quelconque des revendications précédentes, **caractérisé en ce que** l'huile essentielle est préalablement extraite de la lavande stoechade et **en ce que** l'évaporation est réalisée pendant une durée comprise entre 24 et 96 h, sous une pression comprise entre 25 et 75 mbar et à une température comprise entre 40 et 60 °C.

8. Une combinaison susceptible d'être obtenue par le procédé de transformation selon l'une quelconque des revendications 1 à 7, ladite combinaison comprenant une fraction d'huile essentielle et au moins un antibiotique.

9. Une combinaison selon la revendication 8 comprenant une seconde huile essentielle qui a été transformée ou non par le procédé selon l'une quelconque des revendications 1 à 7.

10. La combinaison selon la revendication 8, **caractérisée en ce que** l'huile essentielle est préalablement extraite de la lavande stoechade et l'antibiotique est une pénicilline.

11. La combinaison selon l'une quelconque des revendications 8 à 10, pour une utilisation comme médicament.

12. La combinaison selon l'une quelconque des revendications 8 à 10, pour une utilisation dans la prévention ou le traitement de maladies infectieuses d'origine bactérienne.

13. La combinaison pour l'utilisation selon la revendication 12, **caractérisée en ce que** l'huile essentielle est préalablement extraite de la lavande stoechade et la bactérie est S. *aureus.*

## Patentansprüche

1. Verfahren zur Umwandlung eines ätherischen Öls, umfassend:
- Bereitstellen eines vorab extrahierten ätherischen Öls;
- Verdampfen eines Anteils von zwischen 20 und 50% des ätherischen Öls; und
- Erhalten eines umgewandelten ätherischen Öls, umfassend die Rückgewinnung des nicht verdampften Anteils des ätherischen Öls;
- Kombinieren des nicht verdampften Anteils mit mindestens einem Antibiotikum,
**dadurch gekennzeichnet, dass** die Verdampfung mindestens 2 Std. bei einem Druck von kleiner oder gleich 500 mbar und bei einer Temperatur von zwischen 40 und 100 °C durchgeführt wird, und dadurch, dass das ätherische Öl vorab aus Ajowan, Echter Kamille, Zitronengras, Palmarosa, Schopf-Lavendel, Oregano oder Chinesischem Zimt extrahiert ist.

2. Umwandlungsverfahren nach Anspruch 1, **dadurch gekennzeichnet, dass** die Verdampfung zwischen 30 und 50%, vorzugsweise ungefähr 45% des ätherischen Öls beträgt.

3. Umwandlungsverfahren nach Anspruch 1 oder 2, **dadurch gekennzeichnet, dass** die Verdampfung für eine Dauer von zwischen 18 und 96 Std., noch bevorzugter zwischen 24 und 96 Std., auf noch bevorzugtere Weise für eine Dauer von ungefähr 72 Std. durchgeführt wird.

4. Umwandlungsverfahren nach irgendeinem der vorstehenden Ansprüche, **dadurch gekennzeichnet, dass** die Verdampfung bei einem Druck von kleiner oder gleich 300 mbar, bevorzugt kleiner oder gleich 150 mbar, noch bevorzugter kleiner oder gleich 100 mbar, noch bevorzugter zwischen 25 und 75 mbar, auf noch bevorzugtere Weise bei ungefähr 60 mbar durchgeführt wird.

5. Umwandlungsverfahren nach irgendeinem der vorstehenden Ansprüche, **dadurch gekennzeichnet, dass** die Verdampfung bei einer Temperatur von zwischen 40 und 60 °C, noch bevorzugter bei einer Temperatur von ungefähr 40 °C durchgeführt wird.

6. Umwandlungsverfahren nach irgendeinem der vorstehenden Ansprüche, **dadurch gekennzeichnet, dass** das ätherische Öl vorab aus Schopf-Lavendel oder Oregano extrahiert ist, bevorzugt ist das ätherische Öl vorab aus Schopf-Lavendel extrahiert.

7. Umwandlungsverfahren nach irgendeinem der vorstehenden Ansprüche, **dadurch gekennzeichnet, dass** das ätherische Öl vorab aus Schopf-Lavendel extrahiert ist und dadurch, dass die Verdampfung für eine Dauer von zwischen 24 und 96 Std. bei einem Druck von zwischen 25 und 75 mbar und bei einer Temperatur von zwischen 40 und 60 °C durchgeführt wird.

8. Kombination, die durch das Umwandlungsverfahren nach irgendeinem der Ansprüche 1 bis 7 erhalten werden kann, wobei die Kombination einen Anteil an ätherischem Öl und mindestens ein Antibiotikum umfasst.

9. Kombination nach Anspruch 8, umfassend ein zweites ätherisches Öl, das durch das Umwandlungsverfahren nach einem der Ansprüche 1 bis 7 umgewandelt wurde oder nicht.

10. Kombination nach Anspruch 8, **dadurch gekennzeichnet, dass** das ätherische Öl vorab aus Schopf-Lavendel extrahiert ist und das Antibiotikum ein Penicillin ist.

11. Kombination nach irgendeinem der Ansprüche 8 bis 10, für eine Verwendung als Medikament.

12. Kombination nach irgendeinem der Ansprüche 8 bis 10, für eine Verwendung bei der Vorbeugung oder der Behandlung von infektiösen Erkrankungen mit bakteriellem Ursprung.

13. Kombination zur Verwendung nach Anspruch 12, **dadurch gekennzeichnet, dass** das ätherische Öl vorab aus Schopf-Lavendel extrahiert ist und das Bakterium *S*. *aureus* ist.

## Claims

1. A method for transforming an essential oil comprising:
- supplying a previously extracted essential oil;
- evaporating a fraction of between 20 and 50% of the essential oil; and
- obtaining a transformed essential oil comprising recovering the non-evaporated fraction of the essential oil;
- combining said non-evaporated fraction with at least one antibiotic,
**characterised in that** the evaporation is carried out for at least 2 hours, under a pressure of less than or equal to 500 mbar and at a temperature of between 40 and 100°C, and **in that** the essential oil is previously extracted from ajowan, German chamomile, lemongrass, palmarosa, stoechade lavender, compact oregano or Chinese cinnamon.

2. The transformation method according to claim 1, **characterised in that** the evaporation is between 30 and 50%, preferably approximately 45%, of the essential oil.

3. The transformation method according to claim 1 or 2, **characterised in that** evaporation is carried out for a period of between 18 and 96 hours, even more preferably between 24 and 96 hours, and even more preferably for a period of approximately 72 hours.

4. The transformation method according to any one of the preceding claims, **characterised in that** the evaporation is carried out under a pressure of less than or equal to 300 mbar, preferably less than or equal to 150 mbar, even more preferably less than or equal to 100 mbar, even more preferably between 25 and 75 mbar, even more preferably around 60 mbar.

5. The transformation method according to any one of the preceding claims, **characterised in that** the evaporation is carried out at a temperature of between 40 and 60°C, even more preferably at a temperature of about 40°C.

6. The transformation method according to any one of the preceding claims, **characterised in that** the essential oil is previously extracted from stoechade lavender or compact oregano, preferably the essential oil is previously extracted from stoechade lavender.

7. The transformation method according to any one of the preceding claims, **characterised in that** the essential oil is previously extracted from the stoechade lavender and **in that** the evaporation is carried out for a period of between 24 and 96 h, under a pressure of between 25 and 75 mbar and at a temperature of between 40 and 60°C.

8. A combination obtainable by the transformation method according to any one of claims 1 to 7, said combination comprising an essential oil fraction and at least one antibiotic.

9. The combination according to claim 8 comprising a second essential oil which may or may not have been transformed by the method according to any one of claims 1 to 7.

10. The combination according to claim 8, **characterised in that** the essential oil is previously extracted from stoechade lavender and the antibiotic is a penicillin.

11. The combination according to any one of claims 8 to 10, for use as a medicament.

12. The combination according to any one of claims 8 to 10, for use in the prevention or treatment of infectious diseases of bacterial origin.

13. The combination for use according to claim 12, **characterised in that** the essential oil is previously extracted from stoechade lavender and the bacterium is S. *aureus.*
